# EUROPÄISCHE PATENTSCHRIFT

(11) **EP 1 352 566 B1**
(45) Veröffentlichungstag und Bekanntmachung des Hinweises auf die Patenterteilung: **11.05.2005**
(21) Anmeldenummer: 02405284.7
(22) Anmeldetag: 10.04.2002
(51) Int. Cl.: A23C 9/20, A61K 35/20, A61K 31/35, A61K 9/14, A23K 1/14

(54) **Verfahren zur Herstellung eines Präparates, welches Wirkstoffe aus Cannabis enthält**
Process for the manufacture of a preparation containing active principles from cannabis
Procédé de fabrication d'une préparation de principes actifs à partir de cannabis

(43) Veröffentlichungstag der Anmeldung: 15.10.2003
(73) Patentinhaber: Thomet, Ulrich, 3020 Bern (CH)
(72) Erfinder: Thomet, Ulrich, 3020 Bern (CH)
(74) Vertreter: BOVARD AG - Patentanwälte

(56) Entgegenhaltungen:
- GB-A- 932 378
- G.R. AHMAD ET AL.: "Passive consumption of marijuana theough milk: a low level chronic exposure to delta-9-tetrahydrocannabinol (THC)" JOURNAL OF TOXICOLOGY. CLINICAL TOXICOLOGY., Bd. 28, Nr. 2, 1990, Seiten 255-260, XP008007219 MARCEL DEKKER, NEW YORK, NY., US ISSN: 0731-3810
- S.D. YOO ET AL.: "Mammary excretion of cannabidiol in rabbits after intravenous administration" JOURNAL OF PHARMACY AND PHARMACOLOGY, Bd. 46, Nr. 11, 1994, Seiten 926-928, XP008007226 LONDON, GB ISSN: 0022-3573

## Beschreibung

Die vorliegende Erfindung bezieht sich auf ein Präparat, welches Wirkstoffe aus der Wirkstoffklasse der Cannabioide enthält. Sie bezieht sich insbesondere auf die Produktion einer Milch, welche Cannabioid-Wirkstoffe enthält und auf Produkte, welche aus dieser Milch erhalten werden. Das Verfahren beinhaltet im Wesentlichen natürliche biologische Prozesse, wobei ein Produkt erhalten wird, welches weder chemischen Prozessen noch Extraktions-Prozessen mit Lösungsmitteln unterworfen wurde.

Aus Hanfarten gewonnene Produkte wie Harz oder getrocknete Blätter, Blüten und Knospen werden seit Jahrhunderten als Heilmittel, als Mittel für Kulte und Rituale in asiatischen Kulturen, als Heilmittel und auch als Rauschdrogen verwendet. Die wichtigste Hanfsorte ist Cannabis Sativa, die hauptsächlich in Nord- und Südamerika vorkommt. Weiter gibt es ebenfalls asiatische Hanfsorten, wie Cannabis Indica noch Cannabis Ruderalis. Als Hauptwirkstoff bekannt ist Tetrahydrocannabinol (THC). Dieses Produkt wird in zahlreichen pharmazeutischen Erzeugnissen als Wirkstoff eingesetzt. Der Wirkstoff soll weder eindeutig narkotisierende oder eindeutig stimulierende Wirkungen aufweisen. THC soll sich auf den Serotoninspiegel auswirken, wobei nervöse Zustände entkrampft werden. THC soll ebenfalls bestimmte Gehirnfunktionen stimulieren und halluzinogene Eigenschaften besitzen. THC soll massiv im Körper gespeichert werden und somit auch nach längerer Zeit nach der Einnahme als Stoffwechselprodukte im Körper nachgewiesen werden können. In der chinesischen Medizin wurde der Hanfwirkstoff gegen Rheuma, Malaria und Verstopfung eingesetzt. Auch in Indien wurde Hanf schon vor hunderten von Jahren als Heilmittel und Droge dokumentiert, wobei eine Anwendung für psychosomatische Krankheiten im Vordergrund stand.

Auch in der Patentliteratur gibt es pharmazeutische Präparate, welche Extrakte aus Cannabis beinhalten. Beispielsweise ist in DE 4403157A1 ein schmerzlinderndes Präparat zur topischen Anwendung auf der Haut beschrieben, welches unter anderem ebenfalls einen Extrakt aus Cannabis Sativa (siehe Beispiel 7) enthält. Die Wirkstoffe werden aus den Pflanzen mittels einer Extraktion gewonnen. Im Dokument DE 19757921A1 wird ein Mittel zur Aktivierung des Haarwuchses beschrieben, in welchem getrocknete Pflanzenteile aus Cannabis Sativa verwendet werden.

Es gibt zahlreiche Einsatzgebiete, für welche pharmazeutische Zusammensetzungen, welche Cannabis Komponenten enthalten, eingesetzt werden können: Glaucum Erkrankungen, die mit Appetitlosigkeit verbunden sind, Übelkeit und Brechreiz bei der Chemotherapie, Stress, Migräne, Depression, Asthma, Epilepsie, Krämpfe. Ein weiterer möglicher Einsatz ist die Verwendung als Entgiftungsmittel für Personen, die mit gewissen in die Umwelt freigesetzten Giften kontaminiert wurden.

Die Anwendungsmöglichkeiten von derartigen Produkten sind, neben der Erzeugung eines Rauschzustandes, sehr vielfältig. Neben dem Einsatz von Pflanzenkomponenten werden auch Harz und Pflanzenextrakte von Cannabis eingesetzt. Ebenso wird Cannabis geraucht. Um einerseits eine breite Palette der Wirkstoffe auszunutzen und anderseits eine schädliche Form der Einnahme zu vermeiden sind Verabreichungsformen gefragt, welche optimal verträglich sind.

Es ist Aufgabe der vorliegenden Erfindung, eine Verabreichungsform von Cannabiswirkstoffen zur Verfügung zu stellen, die natürlichen Ursprungs ist, und die für den Konsumenten oder Patienten unschädlich ist. Weiter soll das Produkt weitgehend in Landwirtschaftsbetrieben herstellbar sein.

Es wurde gefunden, dass Milch von Kühen, welche mindestens teilweise mit Hanfpflanzen gefüttert wurden, Cannabiswirkstoffe enthält und demzufolge derartige Milch oder Erzeugnisse davon eine schonende Verabreichungsform für Cannabis-Wirkstoffe darstellt.

Der Gegenstand der vorliegenden Erfindung ist demzufolge ein Verfahren für die Erzeugung von Milch und Milcherzeugnissen, welche einen Gehalt an Cannabinoid-Wirkstoffen aufweisen und die insbesondere als Grundlage von Naturheilmitteln geeignet sind, wobei Milchkühe mit Futter gefüttert werden, welches mindestens teilweise Hanfpflanzen oder Teile davon enthält, und die erhaltene Milch gewonnen und durch mindestens einen technischen Verarbeitungsschritt zu einem haltbaren Milchprodukt weiterverarbeitet wird.

Gemäss einem Aspekt der Erfindung ist der technische Verarbeitungsschritt ausgewählt aus einem der folgenden Schritte: Gefriertrocknen, Trocknen nach dem Sprühverfahren, Bandtrocknung und Fliessbetttrocknung. Dabei wird eine Trockenmilch mit einem Gehalt an Cannabinoid-Wirkstoffen erhalten.

Gemäss einem weitem Aspekt der Erfindung wird die erhaltene Milch durch eine Wärmebehandlung, wie Pasteurisieren, zu einer haltbaren Milch weiterverarbeitet.

Gemäss einem weitem Aspekt der Erfindung werden Hanfpflanzen oder Hanfpflanzenteile, insbesondere Hanfblüten von weiblichen Hanfpflanzen, als Futterzusatz verwendet, die vor Ihrer Verwendung zum Haltbarmachen einer Silage unterworfen wurden.

Gemäss einem weitern Aspekt der Erfindung werden die Kühe mit einem Mischfutter oder Kraftfutter gefüttert, das bei guter Hanfqualität 0,5 bis 3 Gew.% Hanfkomponenten enthält; während es bei geringerer Hanfqualität einen Gehalt von 3 bis 8 Gew.% aufweisen kann.

Gemäss einem weitem Aspekt der Erfindung wird eine Milch erhalten, die einen Gehalt von 0,1 - 5 mg/l, in der Regel 0,5 - 1 mg/l, Δ-9-Tertrahydrocannabinol enthält.

Gemäss einem weitem Aspekt der Erfindung wird die erhaltene Milch zu Joghurt oder Käse verarbeitet.

Gemäss einem weitem Aspekt der Erfindung wird ein haltbares Milchprodukt, das gemäss dem Verfahren der vorliegenden Erfindung erhalten wurde, zur Herstellung eines pharmazeutischen Produktes verwendet, das einen Gehalt an Cannabinoid-Wirkstoffen aufweist. Dazu werden Verfahren angewandt, die zur Herstellung von Naturheilmitteln üblich sind.

Im Verfahren gemäss der vorliegenden Erfindung werden Kühe mit Futter gefüttert, das mindestens teilweise Hanfpflanzen oder Teile davon enthält. Das Hanfmaterial ist vorzugsweise Blüten enthaltendes Material vom oberen Teil der Pflanze. Die Ernte kann mit gleichen Vorrichtungen durchgeführt werden, wie sie für die Maisernte verwendet werden. Durch die Silage erhält man ein Material, das eine bessere Lagerstabilität aufweist als getrocknetes Hanfblütenmaterial. Das übrige Futter kann Silofutter, Trockenfutter oder Frischfutter sein. Vorzugsweise wird das Futter durch einen TMR-Mixer gemischt, um eine Futtermischung zu erhalten, die einen möglichst einheitlichen Gehalt der verschiedenen Bestandteile aufweist. Dabei wird eine Milch erhalten, welche biologisch aktive Wirkstoffe aus der Hanfpflanze enthält. Die Milch kann als Grundstoff für weiterverarbeitete lagerstabile Medizinal-Produkte, insbesondere Naturheilmittel, verwendet werden. Ein interessantes Produkt ist beispielsweise Trockenmilch mit einem Wirkstoffgehalt. Der Δ-9-Tertrahydrocannabinol-Gehalt kann im Bereich von 0,15 mg/l bis 1 mg/l variieren und kann bei optimalen Voraussetzungen bis auf 5 mg/ml gesteigert werden. Die erhaltene Milch kann aber auch andere Wirkstoffe enthalten, die hier nicht explizit erwähnt sind. Die auf diese Weise erhaltene Trockenmilch kann als solche als pharmazeutisches Produkt bereitgestellt werden. Es ist jedoch ebenfalls möglich und sinnvoll, derartige Trockenmilch als Komponente von pharmazeutischen Produkten mit andem Wirkstoffen zu verwenden, sofern dies vom therapeutischen Standpunkt aus sinnvoll ist.

Es wurde festgestellt, dass Kühe, welche zumindest teilweise mit Cannabispflanzen oder Teilen davon in Form von Frischfutter, Trockenfutter oder Silofutter gefüttert werden, sich durchaus wohl fühlen und die Kühe eine Milch mit einem Wirkstoffgehalt abgeben. Die so erhaltene Milch kann weiterverarbeitet werden in Milchprodukte, wie ultrahocherhitzte Milch, Joghurt oder auch Käse, welche einen Wirkstoffgehalt aufweisen, soweit sich die erhaltenen Produkte im Rahmen des Gesetzes des Landes der Produktion bzw. des Konsums bewegen. Ein lagerstabiles Produkt ist Trockenmilch, welche durch Gefriertrocknen von Milch erhalten wird. Neben der Gefriertrocknung kommen als Aufarbeitungsverfahren ebenfalls in Frage: Trocknen nach dem Sprühverfahren, Bandtrocknung und Fliessbetttrocknung. Eine derartige Trockenmilch mit einem Wirkstoffgehalt ist ein idealer Rohstoff für die Herstellung von pharmazeutischen Zusammensetzungen für verschiedene Indikationen entsprechend der obigen Angaben.

Es wird ausdrücklich darauf hingewiesen, dass die gemäss der vorlegende Erfindung erhaltenen Produkte ausschliesslich für die gesetzlich zulässige Verwendung bestimmt sind.

Nachstehend wird die Erfindung anhand eines Beispieles erläutert, wobei dieses Beispiel keine Einschränkung darstellen soll.

### Beispiel:

Eine Kuh mit einer mittleren Milchleistung wird während einer Zeitperiode von zwei Wochen bei einer täglichen Futtermenge von 14 kg/Tag, aufgeteilt in zwei Rationen gefüttert; wobei jeder Ration 50 g Hanfblüten zugegeben wurden. Die verwendeten Hanfblüten bestanden aus dem oberen Teil von weiblichen Hanfpflanzen, die analog wie Mais geerntet wurden. Das Material wurde mit einer Maishäckselmaschine verarbeitet und dann einer Silage unterworfen. Der Rest des Futters war normales Viehfutter ("Total Mix Ratio", TMR), wie es in der Landwirtschaft Kühen für die Milchproduktion abgegeben wird. Die Milch, die eine Woche nach Beginn des genannten Fütterungsplans gewonnen wurde, enthielt einen Wirkstoffanteil an Δ-9-Tertrahydrocannabinol 0,15 mg/l und 0,63 mg/l. Die Messungen wurden in einem akkreditierten Labor (Swiss testing STS 017, EN 45001, GMP) durchgeführt.

In einem Versuch wurden 20 Kilogramm der erhaltenen Milch gefriergetrocknet und zu Milchpulver verarbeitet. Auch hier wurde durch eine Gruppe von Testpersonen festgestellt, dass das erhaltene Milchpulver auf diese die den Cannabinoiden eigene Wirkung ausübt.

## Patentansprüche

1. Verfahren zur Herstellung von Milch und Milchprodukten, die einen Gehalt an Cannabinoid-Wirkstoffen aufweisen und die insbesondere als Grundlage von Naturheilmitteln geeignet sind, **dadurch gekennzeichnet, dass** Milchkühe mit Futter gefüttert werden, welches mindestens teilweise Hanfpflanzen oder Teile davon enthält, und die erhaltene Milch gewonnen und durch mindestens einen technischen Verarbeitungsschritt zu einem haltbaren Milchprodukt weiterverarbeitet wird.

2. Verfahren nach Anspruch 1, zur Herstellung einer Trockenmilch, die einen Gehalt an Cannabinoid-Wirkstoffen aufweist, **dadurch gekennzeichnet, dass** der technische Verarbeitungsschritt, durch welchen die gewonnene Milch zu einem Milchpulver verarbeitet wird, ausgewählt ist aus einem der folgenden Verfahrensschritte: Gefriertrocknen, Trocknen nach dem Sprühverfahren, Bandtrocknung und Fliessbetttrocknung.

3. Verfahren nach Anspruch 1, **dadurch gekennzeichnet, dass** die erhaltene Milch durch eine Wärmebehandlung, wie Pasteurisieren, zu einer haltbaren Milch weiterverarbeitet wird.

4. Verfahren nach einem der Ansprüche 1 bis 3, **dadurch gekennzeichnet, dass** Hanfpflanzen oder Hanfpflanzenteile, insbesondere Hanfblüten von weiblichen Hanfpflanzen, als Futterzusatz verwendet werden, die vor Ihrer Verwendung zum Haltbarmachen einer Silage unterworfen wurden.

5. Verfahren nach einem der Ansprüche 1 bis 4, **dadurch gekennzeichnet, dass** die Kühe mit einem Mischfutter oder Kraftfutter gefüttert werden, das 0,5 bis 3 Gew.% Hanfkomponenten enthält.

6. Verfahren nach einem der Ansprüche 1 bis 5, **dadurch gekennzeichnet, dass** eine Milch erhalten wird, die einen Gehalt von 0,1 - 5 mg/l, in der Regel 0,5 - 1 mg/l, Δ-9-Tertrahydrocannabinol enthält.

7. Verfahren nach Anspruch 1, **dadurch gekennzeichnet, dass** die erhaltene Milch zu Joghurt oder Käse verarbeitet wird.

8. Verwendung eines haltbaren Milchproduktes, das gemäss dem Verfahren nach einem der Ansprüche 1 - 7 erhalten wurde, zur Herstellung eines pharmazeutischen Produktes, das einen Gehalt an Cannabinoid-Wirkstof fen aufweist.

## Claims

1. Method of producing milk and milk products which have a content of cannabinoid active substances and which are particularly suitable as a base for natural medicines, **characterised in that** milk cows are fed feed containing at least in part hemp plants or parts thereof, and the milk is obtained and further processed into a storable milk product through at least one technical processing step.

2. Method according to claim 1, for producing wholly dehydrated milk having a content of cannabinoid active substances, **characterised in that** the technical processing step by which the milk obtained is processed into milk powder is selected from among the following processing steps: lyophilization, drying according to the spray process, horizontal belt drying and fluidized bed drying.

3. Method according to claim 1, **characterised in that** the milk obtained is further processed into a storable milk through a thermal treatment; such as pasteurization.

4. Method according to one of the claims 1 to 3, **characterised in that** hemp plants or hemp plant parts, in particular hemp blossoms of pistillate hemp plants, are used as feed admixture which have been subjected before their use to an ensilage to make them storable.

5. Method according to one of the claims 1 to 4, **characterised in that** the cows are fed with a mixed fodder or concentrated fodder containing 0.5 to 3 % by weight of hemp components.

6. Method according to one of the claims 1 to 5, **characterised in that** a milk is obtained containing a content of 0.1 - 5 mg/l, as a rule 0.5 - 1 mg/l, of Δ-9-tetrahydrocannabinol.

7. Method according to claim 1, **characterised in that** the milk obtained is processed into yoghurt or cheese.

8. Use of a storable milk product which was obtained according to the method of one of the claims 1 to 7, for producing a pharmaceutical product having a content of cannabinoid active substances.

## Revendications

1. Procédé pour la fabrication de lait et de produits laitiers qui présente une teneur en principes actifs à base de cannaboïde et qui sont appropriés en particulier en tant que substance de base pour des remèdes naturels, **caractérisé en ce que** des vaches laitières sont nourries au fourrage qui comprend au moins partiellement du chanvre ou des parties de chanvre et **en ce que** le lait obtenu est issu de la traite et est transformé en un produit laitier de conservation par au moins une étape technique de traitement.

2. Procédé selon la revendication 1, pour la fabrication d'une poudre de lait qui présente une teneur en substances de cannabinoïdes **caractérisé en ce que** l'étape technique de traitement, par laquelle le lait obtenu est transformé en une poudre de lait, est sélectionné à partir de l'une des étapes suivante : séchage par réfrigération, séchage d'après le procédé de pulvérisation, séchage par bande et séchage par lit fluidisé.

3. Procédé selon la revendication 1, **caractérisé en ce que** le lait obtenu est transformé par un traitement thermique, tel que la pasteurisation, en un lait de conversation.

4. Procédé selon l'une des revendications 1 à 3, **caractérisé en ce que** du chanvre ou des parties, en particulier des fleurs de chanvres de plantes de chanvre féminines peuvent être utilisés comme additifs de fourrage, qui ont été soumis à un ensilage avant l'utilisation pour la conservation.

5. Procédé selon l'une des revendications 1 à 4, **caractérisé en ce que** les vaches sont nourries avec un fourrage mixte ou un fourrage concentré qui contient 0,5 à 3 % en poids de composants de chanvre.

6. Procédé selon l'une des revendications 1 à 5, **caractérisé en ce qu'**on obtient un lait comprenant une teneur de 0,1 - 5 mg/l, d'ordre général de 0,5-1 mg/l de Δ-9-tétrahydrocannabinol.

7. Procédé selon la revendication 1, **caractérisé en ce que** le lait obtenu est transformé en yaourt ou fromage.

8. Utilisation d'un produit laitier de conservation qui a été obtenu d'après le procédé selon l'une des revendications 1 - 7, pour la fabrication d'un produit pharmaceutique qui présente des principes actifs de cannabinoïdes
